# EUROPEAN PATENT APPLICATION

(11) **EP 1 757 269 A2**
(43) Date of publication of application: **28.02.2007**
(21) Application number: 06022193.4
(22) Date of filing: 30.07.2002
(51) Int. Cl.: A61K 9/12, A61K 9/14

(54) **Pulmonary formulation comprising silicon particles**

(30) Priority: 01.08.2001 GB 0118689
(62) Divisional of application: 02745691.2
(71) Applicant: pSiMedica Limited, Worcestershire, WR14 3SZ (GB)
(72) Inventor: Canham, Leigh Trevor pSiMedica Limited, Malvern Worcestershire WR14 3SZ (GB); Aston, Roger pSiMedica Limited, Malvern Worcestershire WR14 3SZ (GB)
(74) Representative: Evans, Jacqueline Gail Victoria

(57) **Abstract**

The use of microparticles of silicon and particularly resorbable and/or photoluminescent silicon in the preparation of a medicament for nasal or pulmonary delivery. Aerosol formulations and their preparation are also described and claimed.

These formulations may be used for example as carriers for pharmaceutical compounds as well as having diagnostic applications.

## Description

The present invention relates to the use of microparticles of silicon in formulations, in particular for the nasal or pulmonary delivery. These, and particularly porous silicon microparticles may be used for example in the delivery of pharmaceuticals or therapeutic compounds. It also relates to formulations and in particular to aerosol formulations including these microparticles and to methods of treatment using them.

Administration of variety of substances via pulmonary or nasal delivery is becoming increasingly favoured in a number of contexts. The delivery methods are relatively non-invasive, and frequently do not require specialist assistance at a medical centre for example. They may therefore lead to good patient compliance.

Drugs which are conventionally administered in this way, include compounds such as bronchodilators used in the treatment of conditions such as asthma, chronic obstructive pulmonary disease (COPD) or cystic fibrosis, anti-inflammatories for use in the treatment of allergies such as hayfever. However, antibiotics for use in the treatment of pulmonary infections and lung surfactants for treatment of infant respiratory distress syndrome are also being evaluated.

In addition, it should be noted that pulmonary delivery is a good way of achieving systemic delivery of a range of therapeutic peptides and proteins. This is due to the large surface area of the 300million alveoli (in excess of 30m²) combined with the fact that they have present a thin barrier (0.2 micron thick epithelial lining) to the blood and a low concentration of proteolytic enzymes.

Furthermore, pulmonary or nasal delivery of therapeutics of prophylactic vaccines is being proposed (see for example WO 00/56362). Respiratory mucosae offer certain morphological, physiological and immunological advantages over other non-parenteral sites in terms of immunisation, particularly against pathogenic entities which affect or utilise mucosal surfaces as portals of entry. This is because effective vaccination against these pathogens normally requires mucosae to be adequately protected with locally produced antibodies of the secretory IgA (sIgA) isotype. Whilst mucosal surfaces are usually poorly protected with IgA following parenteral administration of vaccines, it is now apparent that successful delivery of antigenic material to immunoresponsive elements in mucosa-associated lymphoid tissue (MALT) can result in vigorous stimulation of the mucosal arm of the immune system. By means of the common mucosal immune system (CMIS) it is feasible that several anatomically disparate mucosal surfaces could be protected through mucosal administration of a vaccine at a single site. Mucosal vaccination offers the added advantage that some degree of systemic immunity can be induced in concert with local responses due to translocation of antigenic material from sub-epithelial compartments to systemic immunoresponsive tissues such as the spleen.

Drug delivery by inhalation has a long history, with crude inhalers having been used medicinally for at least 200 years. These include simple nebulisers and pressurised metered dose inhalers (MDIs). The basic design of inhalers has been much improved of late with the inclusion of spacer devices to facilitate correct inhalation technique, breath-actuated inhalers. Formulations have included liquid and more recently dry powder formulations.

Propellants such as chlorofluorocarbons (CFCs) which have been used in the past, are being phased out following the Montreal Convention, and dry powder formulations are currently much preferred. These may have high content of active ingredient (e.g. from 50-95%) as compared to aqueous aerosols (1-2%). Also, the risk of microbial growth is lower.

Many of the current lung delivery systems suffer from a number of problems. In particular, in dry powder formulations, particles may agglomerate prior to delivery, making dispersion difficult. Reproducibility is often low, as dosage is heavily dependent on patient's technique.

The respiratory tract itself is a difficult target area. It encompasses the upper airways including the oropharynx and larynx, followed by the lower airways, which include the trachea, leading to bifucations into the bronchi and bronchioles. These together form the conducting airways. The terminal bronchioles then divide into smaller respiratory bronchiole which then lead to even smaller alveolar ducts and sacs of the deep lung. It is the alveoli that are the primary target of inhaled therapeutic aerosols for systemic drug delivery.

The physical dimensions of the respiratory orifices clearly put an upper limit to the penetration of a microparticle, depending upon its size. Whilst the trachea has a diameter of about 2cm, the larger bronchi are between 6 and 12 mm in diameter and these bifurcate 23 times before reaching the furthest recesses of the lung. The respiratory bronchioles have widths of 500-600 microns and the alveolar ducts 400-500 microns. The terminal air saccules, the alveolar chambers, are about 400 microns across. However, in practice, the maximum size of particle, which can remain airborne up to these locations is much lower.

A number of physical, chemical and biological processes determine the fate of particles entering the lungs. Three different physical forces operate within the respiratory system; inertial forces, gravity and diffusion. These constrain the maximum size of particles that can penetrate the various parts of the respiratory tract. As flowing air moves in and out of the lungs, inertial forces within the nasophararyngeal chamber and at the points of branching of the airways, where the direction of flow changes, promote collisions with surfaces. Along the finer airways, particles come into contact with surfaces due to gravitational sedimentation. The third mechanism, which promotes surface uptake of very fine particles in the finest airways, is the diffusional bombardment by gas molecules.

Any insoluble particles contacting the mucus lining of the conducting airways are propelled upwards by means of cilia action towards the pharynx where they are eventually swallowed.

In general therefore, particles typically need to be under 60 microns diameter to even reach the entrance of the bronchial tree, less than 20 microns to reach the terminal bronchioles, and under 6 microns to reach the respiratory bronchioles. Their passage further via the alveolar ducts into the alveolar chambers is possible for solid organic particles having an aerodynamic diameter of under 3 microns, for example from 1-3 microns. The aerodynamic diameter of a spherical particle can be expressed as the product of the geometric diameter and the square root of the particle density. Particles with aerodynamic diameters of 8-10 microns will deposit primarily via inertial impaction in the trachea, and those of 3-5 microns via gravitational deposition in the central airways. Particles of aerodynamic width under 1 micron are mostly exhaled and above 10 microns do not reach the mouth in an efficient manner.

To achieve such properties using pure drug formulations is often difficult and depends very heavily on the physical properties of the drugs involved.

US Patent No. 6,136,295 describes the preparation of aerodynamically light particles for drug delivery to the pulmonary system. These particles may include biodegradable carriers such as polymers, fatty acids or ceramics.

The use of the semiconductor, silicon, in biological applications is described for example in WO 97/06101. Various types of silicon with varying properties are described in this application. In particular, it is described how some forms of porous silicon, in particular mesoporous silicon are resorbable. Resorbable silicon is defined as being silicon which dissolves over a period of time when immersed in simulated body fluid solution.

The applicants have found that microparticles of silicon, and in particular porous silicon, may be efficiently delivered via an aerosol, and may be good carriers for biologically active compounds.

According to the present invention there is provided the use of microparticles of silicon in the preparation of a medicament for nasal or pulmonary delivery. In particular, the microparticles of silicon are resorbable and/or photoluminescent.

Microparticles of silicon of consistent size and shape can be prepared and these have good physical properties for efficient delivery to the respiratory tract.

The term "medicament" as used herein refers to any substance used in therapy or diagnosis. Where the medicament is a substance used in therapy, the silicon microparticles are preferably resorbable, and optionally may also be photoluminescent. For some diagnostic purposes, in particular for tests carried out ex-vivo, or in animal trials, the microparticles are preferably photoluminescent, and more preferably, photoluminescent and resorbable, as outlined hereinafter.

As used herein, the term "resorbable" relates to material which will dissolve in body fluids and in particular lung fluids such as lung intestitial fluids. Such materials will in general be soluble at normal physiological temperatures (37°C ± 1°C) in simulated body fluid, over a period of time, for example of up to 8 weeks, and generally at less than 2 weeks. Simulated body fluid in this case may comprises a solution of reagent grade salts in deionised water so that the ionic concentration reflect that found in human plasma, as shown in the following Table 1, or alternatively it may comprise a simulated lung mucus, and in particular simulated lung interstitial fluid. In simulated body fluid which reflects human plasma, the mixture is buffered to physiological pH values (7.3 ± 0.05), preferably organically, using for example trihydroxymethylaminomethane and hydrochloric acid.

**Table 1**

| Ion | Concentration (mM) | |
|---|---|---|
| | Simulated Body Fluid | Human Plasma |
| Na⁺ | 142.0 | 142.0 |
| K⁺ | 5.0 | 5.0 |
| Mg²⁺ | 1.5 | 1.5 |
| Ca²⁺ | 2.5 | 2.5 |
| HCO₃- | 4.2 | 27.0 |
| HPO₄²⁻ | 1.0 | 1.0 |
| Cr⁻ | 147.8 | 103.0 |
| SO₄²⁻ | 0.5 | 0.5 |

Given the well-known disease of silicosis, when non-biodegradable particles of siliceous material, inhaled into the deep lung, can cause massive fibrosis, the particles used in the present invention are suitably fully biodegradable within an appropriate time span.

Preferably, the resorbable silicon used is silicon which will fully dissolve in simulated lung interstitial fluid as described by Moss (Health Phys Vol 36 March issue pp447-448, 1979) within a period of up to 14 days, preferably up to 10 days, at 37°C. This fluid is prepared as follows:
To 1 litre of deionized water is added in the following sequence,
203.3mg of magnesium chloride hexahydrate
6019 mg of sodium chloride
298.2mg of potassium chloride
142 mg of sodium phosphate dibasic anhydrous
367.6mg of calcium chloride dihydrate
952.6 mg of sodium acetate trihydrate
2604mg of sodium bicarbonate
97mg of sodium citrate dihydrate

In particular, it may be preferable to use highly porous silicon for this purpose.

The microparticles of resorbable silicon used in the context of the invention may be single crystal, polycrystalline (poly-Si) or amorphous silicon.

Preferably they are porous as these particles tend to be more easily resorbed and they may also be used as carriers for various therapeutic or diagnostic materials.

Porous silicon may be classified depending upon the nature of the porosity. Microporous silicon contains pores having a diameter less than 20Å, mesoporous silicon contains pores having a diameter in the range of 20Å to 500Å; and macroporous silicon contains pores having a diameter greater than 500Å. The nature of the porosity of the microparticles of silicon used in the invention may vary depending upon whether nasal or pulmonary delivery is sought, the size and properties of any biologically active agent which are combined with the particles etc.

However, in general for pulmonary delivery, resorbable silicon microparticles are suitably mesoporous silicon.

The silicon may be pure silicon or it may be doped with for example boron. Silicon wafers are classified depending upon the level of doping as either p- or p+. p- wafers have relatively low levels of boron doping, giving rise to high resistitivites for example of 1-3ohm.cm⁻¹. Wafers with higher levels of boron doping are p+ wafers and may have resisitivities for example of 0.005 ohm.cm⁻¹.

Preferably in the present invention, the silicon particles used are derived from p- silicon since these appear to be less stable and more readily resorbed in simulated lung fluid.

The size of the microparticles of silicon used will depend upon the intended mode of application (nasal or pulmonary) and the target area of the respiratory tract, broadly as outlined above. Particles will generally therefore have an average diameter of less than 60microns, in particular an average diameter in the range of from 1 to 20 microns, such as from 1 to 10 microns, and most preferably, for pulmonary delivery, an average diameter of from 1 to 3 microns.

Suitably, the range of sizes amongst a population of particles is small. Preferably, at least 40%, more preferably and at least 70% and most preferably at least 90% of particles have diameters within the ranges specified above in relation to the average diameters.

The pulmonary delivery of silicon particles alone (whether porous or non-porous) may be required in some circumstances. In particular, silicon is relatively opaque to X-rays. Therefore, inhalation of silicon particles to coat the surfaces of the respiratory tract prior to X-ray may be of use in diagnosis. In addition, the visibly fluorescent nature of highly porous silicon may be used to investigate microparticle distribution in vivo after administration by use of bronchoscopes.

Thus the invention further provides a method for detecting particles administered by aerosol formulation, said method comprising including in said formulation photoluminescent silicon microparticles, and thereafter detecting said particles by irradiating them such that they luminesce.

This provides a rapid means of assessing particle trapping with the upper airways, at the stage of animal trials of a pharmaceutical for instance, or to assess the efficacy of targeting of drug loaded particles to particular pulmonary tissue. For instance, the targeting of antimicrobial drug loaded particles to pulmonary infections, or cytotoxic drugs to endobronchial tumours can be monitored. This property may also be used to quantify aerosol density ex-vivo.

Thus in a particular embodiment, the silicon particles used are photoluminescent particles. Where these are used as a medicament in therapy, they will also be resorbable, but for some diagnostic applications, this may not be necessary. It has been noted that particles which are both resorbable and photoluminescent continue to photoluminesce in u.v. light during dissolution in simulated lung interstitial fluid.

However, in particular the particles of the invention are used as a carrier for a biologically active agent. The biologically active agent may be loaded unto the silicon microparticles in various ways. For example, it may be deposited onto the surface of the silicon particles, incorporated into the pores of porous silicon microparticles or the silicon particles which are subsequently converted into microparticles, or it may be bound or otherwise associated with the surface of the silicon.

In particular, the active agent may be dissolved or suspended in a suitable solvent, and the resorbable silicon microparticles may be incubated in the resulting solution for a period of time. Removal of solvent will result in the active substance being deposited on the surface of the microparticles. However, if the microparticles comprise porous silicon, in general, the solution of the active agent will penetrate into the pores of the porous silicon by capillary action, and so after solvent removal, the agent will be present within the pores. Solvent removal may be effected using various methods including freeze drying.

The process of immersion and drying may be repeated more than once in order to achieve the desired loading levels.

If the active agent is a solid but has a sufficiently high vapour pressure at 20°C then it may be sublimed onto the surface of the microparticles.

Alternatively, the active agent may be bound to the surface of the silicon by way of linkers. The linkers will be any group which bonds or becomes associated with the surface of the silicon to make it receptive to bonding to biologically active material, either by covalent, ionic or other bonds such as hydrogen bonds or the like.

The linker may therefore first be attached to the silicon and the biologically active agent then allowed to react with it. Alternatively, the biologically active agent may first be coupled to a suitable linker group, which is then allowed to react with the surface of the silicon.

Examples of suitable linkers are known in the art or will be apparent to a chemist.

For instance, WO 00/26019 describes the derivatisation of porous silicon surfaces by contacting the silicon with an optionally substituted alkene or alkyne and illuminating the surface, for example with a Tungston ELH light. WO 00/66190 describes derivatization of the surface of silicon using methods such as hydrosilyation in the presence of a Lewis acid. In that case, the derivatisation is effected in order to block oxidation of the silicon atoms at the surface and so stabilise the silicon.

Stabilisation of the resorbable microparticles of the invention may be desirable where for example, slow release of biologically active agent in the lung is required. In this case, derivatisation to form Si-C bonds at the surface of the microparticles may be of assistance. This may be achieved by derivatisation with simply alkane or alkenes as described hereinafter. Alternatively, derivatisation may be used to modify the properties of the microparticles such as the hydrophobicity, so as to minimise agglomeration during storage.

However, in particular, the derivatization will be effected in order to covalently bond biologically active agents to the surface.

Thus in a particular embodiment, the microparticles of the invention are pre-treated by reaction with a compound of formula (I) 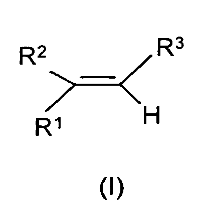 where R¹ is an organic group, which optionally is bound to a biologically active agent or includes an optionally protected functional group which may be bound to a biologically active agent, and R² and R³ are hydrogen, or together form a triple bond, in the presence of a Lewis acid or in the presence of light radiation.

Particular examples of R¹ are hydrocarbon groups which are optionally substituted by functional groups. The term "hydrocarbon" refers to any structure comprising carbon and hydrogen atoms. For example, these may be alkyl, alkenyl, alkynyl, aryl such as phenyl or napthyl, arylalkyl such as benzyl, cycloalkyl, cycloalkenyl or cycloalkynyl. Suitably they will contain up to 20 and preferably up to 10 carbon atoms.

The term "functional group" as used herein refers to reactive groups such as halo, cyano, nitro, oxo, -OC(O)R^{a}, -OR^{a}, -C(O)OR^{a}, S(O)ₜR^{a}, NR^{b}R^{c}, OC(O)NR^{b}R^{c}, C(O)NR^{b}R^{c}, OC(O)NR^{b}R^{c}, -NR⁷C(O)ₙ,R⁶, -NR^{a}CONR^{b}R^{c}, -C=NOR^{a}, -N=CR^{b}R^{c}, S(O)ₜNR^{b}R^{c}, C(S)ₙR^{a}, C(S)OR^{a}, C(S)NR^{b}R^{c} or -NR^{b}S(O)ₜR^{a} where R^{a}, R^{b} and R^{c} are independently selected from hydrogen or optionally substituted hydrocarbyl, or R^{b} and R^{c} together form an optionally substituted ring which optionally contains further heteroatoms such as S(O)ₛ, oxygen and nitrogen, n' is an integer of 1 or 2, s is 0, 1 or 2, t is 0 or an integer of 1-3. In particular the functional groups are groups such as halo, cyano, nitro, oxo, C(O)ₙR^{a}, OR^{a}, S(O)ₜR^{a}, NR^{b}R^{c}, OC(O)NR^{b}R^{c}, C(O)NR^{b}R^{c}, OC(O)NR^{b}R^{c}, -NR⁷C(O)ₙR⁶, -NR^{a}CONR^{b}R^{c}, -NR^{a}CSNR^{b}R^{c}, -C=NOR^{a}, -N=CR^{b}R^{c}, S(O)ₜNR^{b}R^{c}, , or -NR^{b}S(O)ₜR^{a} where R^{a} , R^{b} and R^{c}, n and t are as defined above.

Suitable optional substitutents for hydrocarbyl groups R^{a}, R^{b} and R^{c} are halo, cyano, nitro, oxo, carboxy or alkyl esters thereof, alkoxy, alkoxycarbonyl, amido, mono or di-alkylamido, amino, mono or di-alkylamino, alkyl sulphonyl, or thioalkyl.

In particular, R¹ will comprise a hydrocarbon group which is substituted by at least one functional group, which will allow subsequent coupling bonding to a biologically active agent. For example, where R¹ includes a substituent which is a leaving group such as halo, mesylate or tosylate, subsequent binding to a biologically active agent using a conventional nucleophilic coupling reaction in the presence of a base may be effected. If necessary, however the functional group may be protected during the hydrosilylation reaction. Suitable protecting groups would be apparent to a skilled chemist, but for example acid groups may be esterified and hydroxy groups etherified.

Alternatively, a functional group which is not reactive under the conditions used in the hydrosilylation reaction may be used, and subsequently changed to a different functional group using conventional chemical methods.

Conditions under which such reactions can be effected are described for example in WO 00/26019 and WO 00/66190. For example, when a Lewis acid such as an alkyl aluminium chloride such as ethyl aluminium chloride is used, this is preferably first dissolved in hexane and the resultant solution brought into contact with the microparticles of resorbable silicon under an inert atmosphere for example of nitrogen, together with the compound of formula (I). The reaction may be allowed to continue at moderate temperatures and conveniently at ambient temperature for a period of about an hour, wherein the reaction can be quenched using tetrahydrofuran, followed by dichloromethane. The microparticles may then be washed in ethanol and dried in a stream of nitrogen gas.

Biologically active agents which may be bound to the surface include the pharmaceuticals etc. listed above. In addition however, they may include targeting moieties such as antibodies or binding fragments thereof, which may specifically or non-specifially target particular sites within the lung. For example, the particles carrying cytotoxins may be targeted specifically to tumour cells within the lung, using antibodies or binding fragments which are specific for tumour cell epitopes. Similarly, particles which are loaded with antimicrobial agents may be derivatised using moieties which specifically bind the target microbe.

In particular, the silicon particles used in the invention, prior to loading with any biologically active agent have a tap density of the silicon particles of less than about 0.4 g/cm³, more preferably of less that 0.2g/cm³. Tap density is a standard measure of the mass of the particle divided by the envelope volume within which is can be enclosed. The tap density of particles may be measure using for example a GeoPyc. TM (Micrometrics Instrument Corp., Norcross, Ga 30093).

The biological active agent used in the invention may be any pharmaceutically or diagnostically useful compound, including proteins such as antibodies, peptides and genetic constructs such as DNA, RNA or plasmids or vectors. Particular examples include bronchodilators, antibacterial compounds, genetic material including gene therapy vectors, radioactive materials, vaccines include DNA vaccines and sub-unit proteins or peptides, hormones such as insulin, erthropoietin, calcitonin and growth hormones, cytokines such as interferons and interleukins, and anti-cancer compounds including cytotoxins.

In particular however, the biologically active agent will comprise a bronchodilator such as albuterol, bitolterol, salmeterol, ipratropium, fluticasone, clenbuterol, ephedrine, and terbutaline.

Calcification processes, in which calcium phosphate layers deposit on the silicon in a biological environment, would be undesirable in the lung, unless the calcium phosphate phase itself was eventually resorbable. In addition to the biologically active agent, an anti-calcification reagent may be combined with the silicon microparticles, and in particular may be incorporated into porous silicon particles, in a similar manner to the biologically active agents. Suitably, these agents are introduced into the porous particles at the final stage of preparation via solution infiltration and solvent removal.

There has been a lot of research on such agents for cardiovascular biomaterials where calcification is a common and important problem (see for example the review by Levy et al in Biomaterials 12,707-714(1991) and the US patent 5,697,972). Suitable chemical agents include aluminium, iron, magnesium and zinc ions (for example in the form of pharmaceutically acceptable salts), phosphonates, citrate, high levels of fluoride ions, dimethylsulfoxide, sodium dodecyl sulfate, amino acids, polyacrylic acid, and metallocene dichlorides.

Compositions for administration by inhalation may be in the form of a conventional pressurised aerosol arranged to dispense the silicon microparticles as an aerosol containing finely divided solid. Thus in a further aspect, the invention provides an aerosol composition comprising microparticles of resorbable silicon optionally including a biologically active agent as described above.

The composition may include further conventional aerosol components such as propellants and dispersants. Conventional aerosol propellants such as volatile fluorinated hydrocarbons or hydrocarbons as well as dispersants as known in the art, may be used. However, preferably, the compositions are in the form of a dry powder.

The compositions of the invention are suitably included in an aerosol device which is preferably arranged to dispense a metered quantity of active composition.

Compositions for administration by insufflation may be in the form of a finely divided powder comprising microparticles of silicon of average diameter of, for example, 30microns or less. The powder may comprise resorbable silicon alone optionally loaded with one or more active ingredients as described above, or mixtures of these resorbable silicon powders mixed with active ingredients also in the form of powders. The powder for insufflation is then conveniently retained in a capsule containing, for example, 1 to 50mg of powder for use with a turbo-inhaler device, such as is used for insufflation of the known agent sodium cromoglycate. These capsules form a further aspect of the invention.

Microparticles of silicon for use in the invention may be prepared in various ways. For example, they may be prepared using photolithography followed by isotropic chemical etching and then stain etching. Silicon microparticles having particularly high porosity for use in aerosols may require specific drying techniques, such as supercritical drying (see for example USP 5,914,183), freeze drying or pentane drying to prevent collapse of the skeleton as the etching solution is removed.

In particular, a silicon on insulator (SOI) wafer may be photolithographically etched by standard wet etch or dry etch techniques such as those described in PCT/GB99/02381. The etch may be performed in such a manner that an array of silicon microparticles are formed on the oxide substrate. The microparticles may have dimensions in the range 10 to 250 µm. The microparticles can be detached from the oxide substrate by standard HF soak. The microparticles can then filtered off, washed and dried prior to porosification. In this way a particulate product comprising porous silicon particles of monodispersed size and shape may be obtained. These particles can then be isotropically etched down in size and porosified by suitable wet etches.

Porosification of the above silicon particles to achieve particles having the desired tap density may be achieved by standard stain etching as described in J Applied Physics 78(6) p4273-4275 (1995), or light-assisted stain etching as described in Physical Chemistry Chemical Physics 2(2):277-281, 2000. The lithographically based approach allows the fabrication of silicon particles having a well defined shape and narrow size distribution.

The use of a stain etch may not only cause porosification of the sample of silicon to which it is applied, but it may also dissolve at least some of the porous silicon that is so formed. As a result, the particle sizes can be reduced to the levels preferred for use in the present invention, as illustrated hereinafter. The etching conditions can be modified in accordance with the diameter, shape and tap density of porous particle required. In particular, the applicants have found that etching may be readily achieved, generally in one step, using combination of HF and nitric acid, and preferably a combination of 40wt% HF with 70% nitric acid.

Alternatively, particles with very low tap densities may be achieved by producing silicon microparticles with a hollow core. This can be achieved by deposition of silicon on a monodisperse sacrificial core material, followed by transformation of the shell material.

For instance, silicon particles may also be fabricated using polycrystalline silicon. A layer of phosphosilicate glass (PSG) may be deposited on a silicon substrate. The deposition may be performed using atmospheric pressure CVD by reacting pure silane and phosphine with oxygen in a nitrogen stream. The PSG is then patterned by conventional techniques to form an array of base structures. A layer of polycrystalline silicon can then deposited by pyrolysis of silane using low pressure CVD. The polycrystalline silicon layer is then patterned, by standard etching techniques, in such a manner that each base structure is enveloped in an island layer of polycrystalline silicon, and that the island layer is also bonded to the silicon substrate adjacent to the base structure. Heating the polysilicon layer to temperatures between 950 and 1100C for 10 to 30 minutes causes the polysilicon layer to deform as a result of the release of P₂O₅ from the PSG. By selecting the correct form of patterning and conditions the detached silicon particles comprising shell like structures may be used for microparticles for use in the invention.

In a particularly preferred embodiment, particles of silicon of the requisite size are produced by grinding silicon powders, and particularly porous silicon powders between wafers of crystalline silicon. Since porous silicon has lower hardness than bulk crystalline silicon, and crystalline silicon wafers have ultrapure, ultrasmooth surfaces, a Silicon wafer/porous Si powder/Silicon wafer sandwich is a convenient means of achieving for instance, a 1-10 micron particle size from much larger porous silicon particles derived for example, via anodisation. Thus this provides a good method for reducing the size of silicon particles, down to the required levels for use in the context of the present invention. Where the silicon particles are porous and the presence of biologically active material is required, this may be loaded into porous particles either before or after the grinding process. This method forms a further aspect of the invention.

In yet a further aspect, the invention provides a method of delivering a therapeutic or diagnostic agent to a patient in need thereof, said method comprising delivering to the patient a composition as described above.

The invention will now be particularly described by way of example, with reference to the accompanying diagrammatic drawings in which:
Figure 1 shows images of varying degrees of magnification of silicon particles produced in accordance with a method of the invention; and
Figure 2 shows images of particles obtained using a photolithographic dry etching and stain etching procedure.
Figure 3 shows cross-sectional images of a submicron porous silicon film as a function of time in simulated lung fluid (SLF);
Figure 4 shows similar images for a 2.5 micron thick film of the same porosity, made in the same type of wafer, at the same current density, and in the same electrolyte;
Figure 5 shows the results of incubation of a 10.7micron high porosity (80%) silicon layer with a high porosity (80%) in simulated lung fluid;

### Example 1

A sample of 95% porosity silicon microparticles having a tap density of the order of 0.1165g/cm³ can be prepared. A 20 to 30 Ωcm p type (100) silicon wafer with a 10 micron thick p++ top layer is coated on both sides with 100 nm of silicon oxide. The silica layer on the back of the wafer is then patterned with a membrane photomask and reactive ion etched to define the wafer area to be thinned. A supported 10 micron thick membrane is then realised by wet etching through from the back of the wafer to the p++/p- interface. For a 475 micron thick wafer and KOH at 80C this takes 10 to 15 hours. Thick photoresist is then deposited in the back etched cavity as a support for the membrane and as a substrate from which the silicon particles may be removed. Positive photoresist is spun on the front face of the wafer and pattered with a photomask containing thousands of 10x10 micron spaced squares. The silica and p++ membrane are then reactive ion etched. The thick photoresist/diced silicon membrane is then removed from the wafer and placed in a centrifuge tube. The silicon cubes can then be released by dissolving the photoresist in acetone, and collected by centrifugation. Microparticles of the desired size are then separated.

These can then be porosified to the target level using stain etch methods. A stain etch solution comprising hydrofluoric acid and nitric acid may be employed. The stain etch solution is formed by combining 100 volumes of 40% aqueous hydrofluoric acid solution with 1 volume of 70% aqueous nitric acid solution; this stain etch solution will be referred to as the "100:1 solution". The 100:1 solution may be applied to the particulate product for a period sufficient to yield silicon particles having the desired level of porosity. The stain-etched microparticles of high porosity are dried with the aid of supercritical fluid technology as described in Canham et al., Nature, 368, 133-135 (1994) and US patent 5,914,183.

The particles thus produced may be used in the formation of pharmaceutical compositions as described above.

### Example 2

Microparticles of very high (80-95%) porosity can also be prepared by anodization of photolithographically patterned wafers by standard methods such as that described in US 5,348,618. A 20 to 30 ohm cm p type (100) wafer with a 10 micron thick 0.01 ohm cm epitaxial p++ top layer is first photopatterned and dry etched into an array of 10 micron diameter protruding p++ posts. The photoresist from the tops of the posts is then removed and the structure planarized by spin-coating with a low viscosity electrically insulating material. A brief second dry etch step then re-exposes the top of the silicon posts, in preparation for anodisation in 10% ethanoic HF. This is conducted at current densities in the range 50 to 500mA/cm², depending on the particle porosity required. Once the pore front has progressed through the p++ structures into the underlying p- wafer, the current density is changed to a value that will porosify isotropically to a further distance of at least 5 microns. The current density is then ramped up to initiate electropolishing and thereby lift-off a membrane containing porous cylindrical particles and insulating material on one side, and porous p-layer on the other. The more chemically reactive porous layer is then selectively removed in either dilute alkali or an HF-based solution. The monodisperse porous cylinders are then released by dissolving the insulating organic in a suitable solvent.

### Example 3

### Anodisation and porous silicon (pSi) powder/crystalline silicon (c-Si) wafer grinding

A 0.005 ohm cm p-type wafer was anodised for 20 minutes at 25mA/cm2 in equal volumes of 40% HF and ethanol. It was then given a much higher burst of current (150mA/cm2 for 30 seconds) to create a high porosity, structurally weak attachment to the underlying wafer. Upon wafer removal from the electrolyte, rinse in ethanol, and air dry, the latter thin layer disintegrated, releasing the first layer as large flakes. These were collected and placed on the upper polished surface of an unanodised wafer of the same origin.

A second unanodised wafer with its highly polished surface face down was then placed on top of the first wafer. The two wafers were then rubbed against one another using light hand presssure in a figure of eight motion. Due to their smoothness, the two wafer surfaces progressively mated together better and better, and effectively trapped the pSi ultrafine powder, as the pSi particles were reduced in size. After 5 minutes of grinding, the wafer sandwich was placed in ethanol which facilitated porous silicon particle removal and collection from their surfaces.

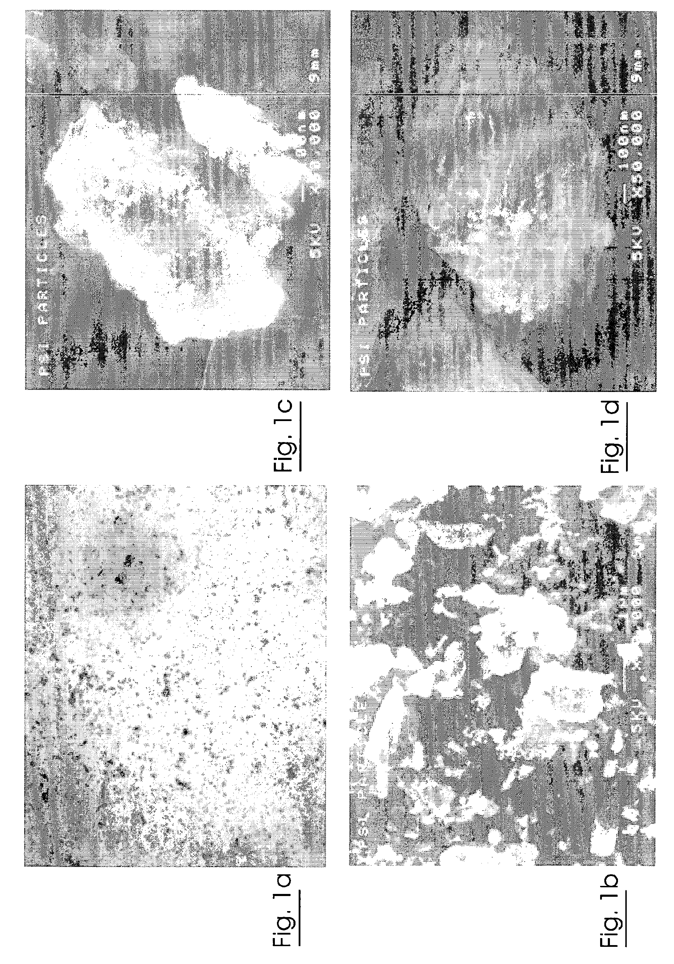

The ethanol /pSi suspension was light brown in colour and was allowed to stand for 24 hours so that any unwanted particles of more than 10 micron diameter would be removed by gravitational settling. The supernatant was then collected and following ethanol evaporation, examined by optical and electron microscopy. Figure 1(a) is an optical image at x500 revealing all particles to be less than 10 micron width. Figure 1(b) is an SEM image at x10,000 revealing the varied shapes of the particles. Figure 1(c) and 1(d) are very high magnification images (x50,000) demonstrating that ultrasmall particles have retained their porous nature.

### Example 4

### Photolithographic dry etching and stain etching

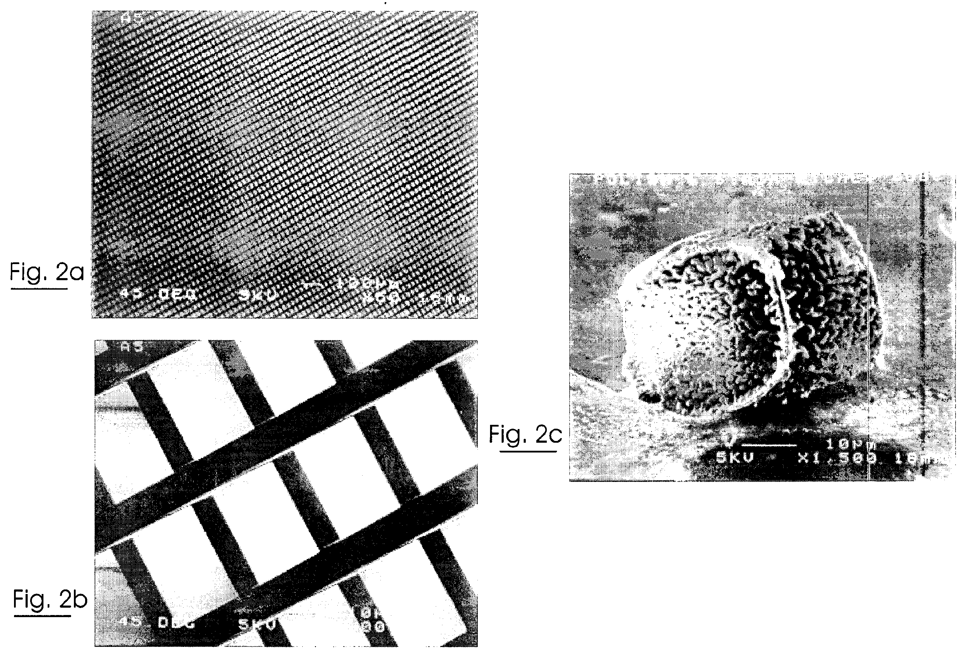

Silicon On Insulator (SOI) wafers with a 30 micron thick Si layer were patterned using a 30 micron square optical mask and HPR-505 photoresist of thickness 1.55 micron, and then dry etched for 24 minutes down to the oxide layer. This generated the array of 30 micron cubes shown in figure 2(a,b). These particles were then released from the wafer by immersion in HF which dissolves the underlying oxide support. Further size reduction, rounding of corners and porosification is then achieved via stain etching in a solution containing HF,nitric acid and water. Figure 2(c) shows an example of a 100 micron perfect silicon cube that has been greatly reduced in size and porosified in one etching step using a 50 to 1 volume ratio of 40wt% HF to 70% nitric acid.

### Example 5

### Stability of porous silicon in simulated lung fluid

Segments of anodised wafers containing thin surface films of porous silicon were incubated for times ranging from 1hour to 10 days at 37°C in sealed polypropylene bottles, completed filled with the simulated insterstitial lung fluid prepared as described above. The pH of the solution was monitored and remained in the range 7.4-7.6 at 37°C.

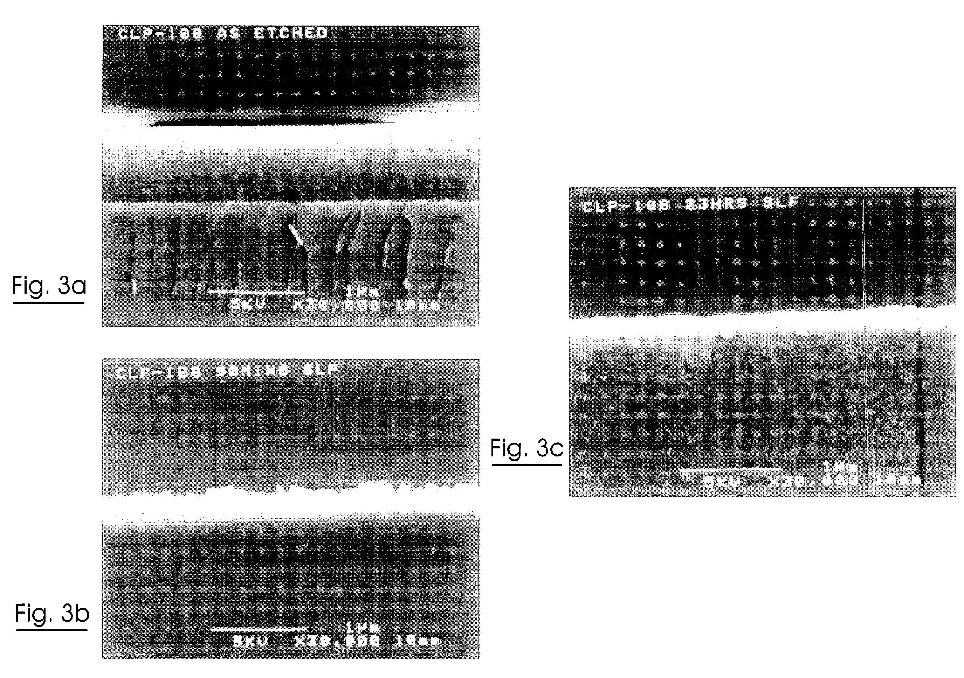

Figure 3 shows cross-sectional images of a submicron porous film as a function of time in simulated lung fluid (SLF). The 0.76 micron thick film of 80% porosity (Figure 3(a)) was made by anodisation of a 1-3 ohm cm resistivity p-type Si wafer at 20mA/cm2 for 1 minute in equal volumes of 40%HF and ethanol. After only 90 minutes in SLF (Figure 3(b)), about 90% of the film has dissolved leaving a residual rough porous layer of about 0.1 micron. Within a day (Figure 3(c)) all the porous silicon has been converted to silicic acid, the narrow white layer evident of about 0.03 micron arising from the oxidised bulk silicon support wafer.

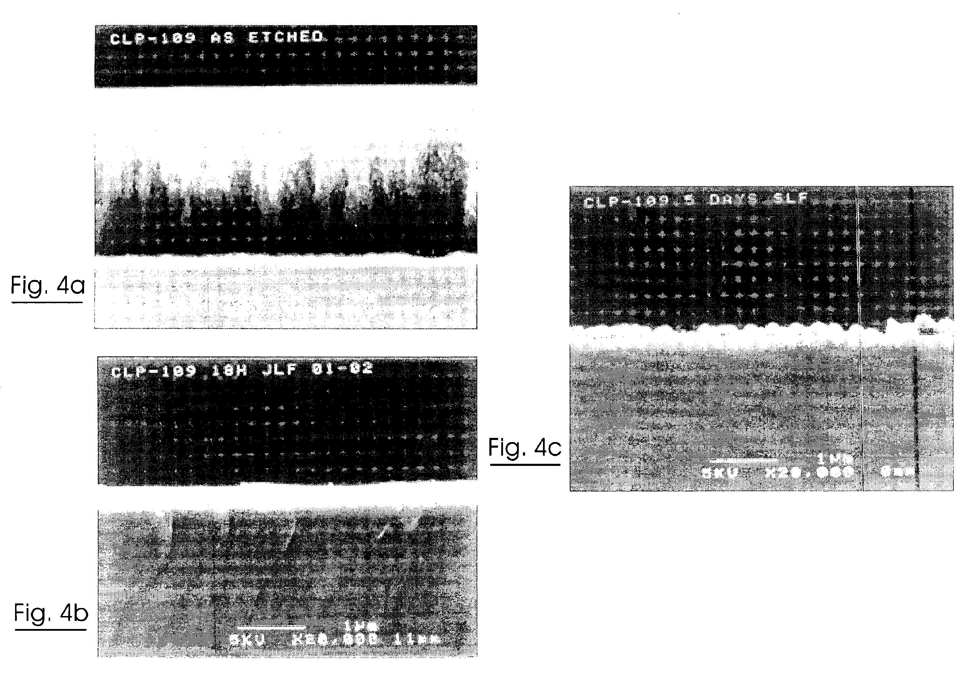

Figure 4 shows similar images for a 2.5 micron thick film of the same porosity, made in the same type of wafer , at the same current density, and in the same electrolyte. The anodisation time was now 4 minutes. Figure 4(b) reveals that about 90% dissolution takes about 18 hours in this case. Comparison of Figures 3 and 4 suggests that the time for complete biodegradation does not scale linearly with porous silicon film thickness for a given microstructure. This implies that a 10 micron diameter particle may take substantially more than double the time for a 5 micron particle to dissolve.

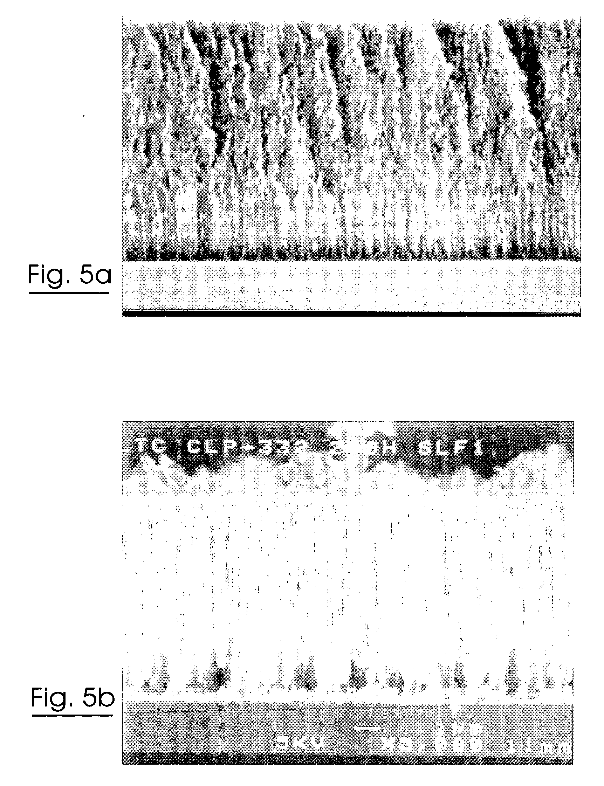

In addition,mesoporous silicon derived from p+ rather than p-wafers was found to have much higher stability. Indeed, the much thicker (10.7micron) but high porosity(80%) layer shown in Figure 5(a) was found to have lost only 20% of its thickness over about 10 days of incubation. Such structures, whilst undergoing corrosion throughout their thickness, were also stable enough to nucleate and support a calcium phosphate overlayer (Figure 5(b)).

## Claims

1. The use of microparticles of silicon in the preparation of a medicament for nasal or pulmonary delivery.

2. The use according to claim 1 wherein the microparticles are resorbable or photoluminescent silicon in the preparation of a medicament for nasal or pulmonary delivery.

3. The use according to claim 2 wherein the resorbable silicon is porous silicon, preferably mesoporous silicon.

4. The use according to claim 3 wherein a biologically active agent is incorporated into the pores of the silicon.

5. The use according to any one of the preceding claims wherein the microparticles of silicon are derived from a p-silicon wafer.

6. The use according to any one of the preceding claims wherein the surface of the silicon is derivatised, preferably to increase the stability or hydrophobicity of the microparticles.

7. The use according to claim 6 wherein a biologically active agent, preferably comprising a targeting moiety which specifically binds a target cell in the respiratory tract, is bound to the surface of the silicon by way of a linker group.

8. The use according to claim 4 or claim 7 wherein the biologically active agent is selected from a pharmaceutically or diagnostically useful compound, preferably a bronchodilator, antibacterial compound, genetic material, radioactive material, vaccine, hormone, cytokine or anti-cancer compound.

9. The use according to any one of the preceding claims wherein the silicon particles have an average diameter in the range of from 1 to 20 microns, preferably of from 1 to 10 microns.

10. The use according to any one of the preceding claims wherein the tap density of the silicon microparticles is less than 0.4g/cm³.

11. The use according to any one of the preceding claims wherein the silicon particles further comprise an anti-calcification agent.

12. An aerosol formulation comprising particles of resorbable and/or photoluminescent silicon optionally including a biologically active agent.

13. An aerosol formulation according to claim 12 wherein the particles of silicon are porous.

14. An aerosol formulation according to claim 13 in the form of a dry powder.

15. An aerosol delivery device comprising a formulation according to any one of claims 12 to 14.

16. A device according to claim 15 wherein the device is arranged to deliver a metered dose of the formulation.

17. A capsule for delivery by insufflation using a turbo-inhaler, comprising resorbable silicon microparticles as defined in any one of claims 1 to 11.

18. A method of preparing silicon particles for use in the preparation of a medicament for nasal or pulmonary delivery which comprises grinding a silicon powder, preferably a porous silicon powder, between the surfaces of crystalline silicon wafers.

19. A method according to claim 18 wherein the silicon powder is loaded with a biologically active agent before grinding.

20. A method according to claims 18 or 19 wherein the silicon powder is obtained by anodisation of a silicon wafer, preferably a p-wafer.

21. A method according to any one of claims 18 to 20 wherein the silicon particles obtained have an average diameter of from 1 to 10 microns.

22. A method for detecting particles administered by aerosol formulation, said method comprising including in said formulation photoluminescent silicon microparticles, and thereafter detecting said particles by irradiating them such that they luminesce.

23. A method according to claim 22 wherein the particles are administered to the upper airways of an animal, and they are detected using a bronchoscope illuminated with u.v. light.

24. A method according to claim 22 wherein which is used to assess the efficacy of targeting of drug loaded silicon particles to particular pulmonary tissue.

25. A method according to claim 22 for quantifying aerosol density ex-vivo.

26. A method for conducting X-ray diagnosis of respiratory tract, said method comprising administering silicon particles to coat the surfaces of the respiratory tract of a patient prior to X-ray.
